# EUROPÄISCHE PATENTSCHRIFT

(11) **EP 1 755 531 B1**
(45) Veröffentlichungstag und Bekanntmachung des Hinweises auf die Patenterteilung: **29.07.2009**
(21) Anmeldenummer: 05740056.6
(22) Anmeldetag: 27.04.2005
(51) Int. Cl.: A61Q 15/00, A61K 8/26, A61K 8/28, A61K 8/365

(54) **WÄSSRIGE ANTITRANSPIRANT FORMULIERUNG**
AQUEOUS ANTI-PERSPIRATION FORMULATION
FORMULATION ANTI-TRANSPIRATION AQUEUSE

(30) Priorität: 27.04.2004 DE 102004020711; 13.04.2005 DE 102005017032
(43) Veröffentlichungstag der Anmeldung: 28.02.2007
(73) Patentinhaber: Beiersdorf AG, 20253 Hamburg (DE)
(72) Erfinder: SCHULZ, Ulrike, 20253 Hamburg (DE); TERSTEGEN, Lara, 20253 Hamburg (DE); ZWIENER, Torben, 22529 Hamburg (DE); CIERPISZ, Yvonne, 20251 Hamburg (DE); ENGFELDT, Linda, 22047 Hamburg (DE); MIERTSCH, Heike, 22529 Hamburg (DE); NÜBEL, Thomas, 22525 Hamburg (DE); ROHDE, Claudia, 25499 Tangstedt (DE); URBAN, Michael, 22393 Hamburg (DE); CHRIST, Gordon, 22763 Hamburg (DE); WARNKE, Katja, 20257 Hamburg (DE); DIEC, Khiet Hien, 20251 Hamburg (DE)
(86) Internationale Anmeldenummer: PCT/EP2005/051894
(87) Internationale Veröffentlichungsnummer: WO 2005/105027

(56) Entgegenhaltungen:
- WO-A-94/12115
- WO-A-97/06777
- DE-A1- 10 237 054
- DE-A1- 19 962 881
- US-A- 6 042 816

## Beschreibung

Die Erfindung betrifft eine wässrige kosmetische Antitranspirant Formulierung mit erhöhter Antitranspirant Wirksamkeit.

Vor allem aus ästhetischen Gründen werden transparente und transluzente Produkte von vielen Verbrauchern bevorzugt. Transparente Formulierungen kommen so z. B. häufig als Deo oder Antitranspirant (AT) zum Einsatz. Diese lassen sich heutzutage durch folgende Technologien realisieren:
1. wässrig-alkoholische Formulierungen
2. Wasser-in-Silikon-Emulsionen
3. Mikro-Emulsionen

Die wässrig alkoholischen Deo- und AT-Formulierungen basieren zumeist auf Wasser und Alkohol als Medium, Deo- und Antitranspirantmittel als Wirkstoffe sowie Parfüm, Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Nachteilen behaftet. So ist beispielsweise die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hocheffektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichend großen Ölphase kann dieser allerdings nicht kaschiert werden. Darüber hinaus führt die Verwendung von Kohlenhydrat-Verdickem zu einer hohen Klebrigkeit des Produktes nach dem Verdunsten des Alkohols.

Wasser-in-Silikon-Emulsionen gehören zur Gruppe der Wasser-in-Öl-Emulsionen. Die Wasserphase, enthaltend Ethanol oder mehrwertige Alkohole wie beispielsweise Propylen Glycol und wasserlösliche Wirkstoffe wie AT-Mittel und/oder Deowirker, nimmt etwa 75-90% der Formulierung ein. Die Ölphase besteht aus einem flüchtigem und einem nicht-flüchtigen Silikonöl sowie einem Silikonemulgator.

Die Transparenz von Wasser-in-Silikon-Emulsionen basiert auf Angleichung der Brechungsindices beider Phasen. Nachteilig ist, dass schon eine z.B. durch Verdunstung bedingte Abweichung der Indices um 0.0004 zu Eintrübungen führt. WO 98/32418 und WO 92/05767 beschreiben derartige Deo- bzw. AT-Formulierungen auf W/Si-Emulsionsbasis.

Ein Ansatz zur Lösung der geschilderten Nachteile ist durch kosmetisch ansprechende alkoholfreie und transparente Produkte möglich geworden, die auf so genannten Mikroemulsionen basieren. Diese haben den großen Vorteil, dass man auch größere Mengen an verschiedenen Ölen - mit all den beschriebenen positiven Effekten für den Verbraucher - stabil einarbeiten kann. Formulierungen dieser Art sind prinzipiell mittels Phaseninversionstemperatur-Technologie (PIT) oder Hochdruckhomogenisierung zugänglich. Die notwendige Stabilität des Emulgatorsystems gegenüber hohen Konzentrationen an Antitranspirantsalzen stellt jedoch hohe Anforderungen an die Formulierungskunst des Produktentwicklers.

WO 98/15255 beschreibt Mikroemulsionen. Nachteilig ist jedoch auch bei diesen Formulierungen ein durch den Verdicker bedingtes klebriges Hautgefühl und eine fehlende Fließgrenze.

Aufgabe der vorliegenden Erfindung ist es, eine kosmetische Zubereitung bereit zu stellen, die den Stand der Technik bereichert und deren Nachteile vermeiden hilft.

Insbesondere ist es die Aufgabe der vorliegenden Erfindung eine kosmetische Formulierungen bereit zu stellen, die transparent ist und sich durch eine minimierte Klebrigkeit auszeichnet. Insbesondere bestand die Aufgabe darin eine Antitranspirantformulierung bereit zu stellen, die transparent ist und keinerlei Eintrübung aufweist, die sich durch eine minimierte Klebrigkeit auszeichnet und die eine definierte Fließgrenze zur optimierten Ausbringung und Applikation besitzt.

Zur Erhöhung der Antitranspirantwirksamkeit ist es üblich, die Menge an Antitranspirantwirkstoff, wie beispielsweise Aluminiumchlorohydrate ACH, zu erhöhen. Des weiteren sind sog. aktivierte Aluminiumchlorohydrate (AACH) als Antitranspirant-Wirkstoffe mit erhöhter Wirksamkeit bekannt, z.B. EP 925783 oder in der Literatur - Antitranspirants and Deodorants, 2nd Edtion, Cosmetic and Technology Science, Vol. 20. 1999.

Problem dabei ist aber, dass die Antitranspirant Wirksamkeit durch eine Erhöhung der Wirkstoffmenge nur begrenzt möglich ist, da sich ab einem Anteil von ca. 15 Gew.% des AT-Wirkstoffes eine Sättigung der Wirksamkeit einstellt und zudem Nachteile wie weiße Rückstände und unangenehmes Hautgefühl verstärkt werden.

Dazu einige chemische Grundlagen:
Löst man Aluminiumsalz AlX₃ einer starken Säure (z.B. AlCl₃) in Wasser, so bildet sich gemäß der Reaktion:

   AlX₃ + 6H₂O→ Al(H₂O)₆³⁺ + 3X⁻

   das oktaedrisch gebaute Hexaaquaaluminium-lon [Al(H₂O)₆]³⁺, das als schwache Kationensäure wirkt.

Als Folge der Säurewirkung unterliegen diese der Hydrolyse und können sukzessiv bis zum Hexahydroxoaluminat-Ion [Al(OH)₆]³⁺ deprotoniert werden.

Abhängig vom pH-Wert und der Konzentration von Aluminiumionen bilden sich dreidimensionale Strukturen durch Verbrückung mit Hydroxidionen und Sauerstoffatomen aus. Diese Vorgänge, bei denen Verbrückungen von Elementatomen mit Hydroxidionen erfolgen, werden Olation genannt und bei Verbrückungen mit Oxid-Ionen spricht man von den Oxolationen.

Beide Reaktionen gehören zu der Gruppe der Kondensationsreaktionen.

Die in wässrigen Aluminiumsalzlösungen vorliegenden mehrkernigen Aluminium-Kationen (Alₘ(OH)ₙ(H₂O)ₒ]^{p+} zählen zur Gruppe der Isopolyoxo-Kationen.

Um eine erhöhte Antitranspirant Wirksamkeit klassischer Aluminiumchlorohydrat (ACH)-Lösungen zu erreichen, werden diese abhängig von Konzentration, Temperatur und Druck thermisch behandelt und die resultierenden Lösungen werden mittels Sprühtrocknung getrocknet.

Damit wird erreicht, dass eine erhöhte Menge an kleineren Molekülgrößen stabil vorliegt. Diese als Antitranspirant wirksame aktivierten Aluminium-komplexsalze (AACH) zerfallen aber in Wasser in ihren ursprünglichen Gleichgewichtszustand zurück, so dass in wässrigen Zubereitungen eine erhöhte Wirksamkeit verloren geht.

Ein Einsatz dieser aktivierten ACH-Typen (AACH) macht bislang daher nur in nichtwäßrigen Systemen einen Sinn, da sonst eine Rückbildung zu der Molekülgrößenverteilung wie sie in klassischen ACH-Lösungen vorkommt, möglich ist, wie beispielsweise in dem Artikel von A. H. Rosenberg - Antitranspirant Technology. SÖFW-Journal, 128 (4) 2000, beschrieben.

WO-A-9412115 offenbart kosmetische Antitranspirantien enthaltend antitranspirant wirksame aktivierte Aluminiumverbindung (Aluminium-Zirkonium Tetrachlorohydrex Glyzin Verbindung), Wasser und Milchsäure.

US-A-6042816 beschreibt wässrige kosmetische Formulierungen umfassend neben antitranspirant wirksame aktivierte Aluminiumverbindungen Wasser und Glykolsäure. Formulierungen der WO-A-9412115 und US-A-6042816 sind als Antitranspirantien zu verwenden.

Aufgabe der vorliegenden Erfindung ist es daher eine wässrige Zubereitung zur Verfügung zu stellen, die eine erhöhte Antitranspirant Wirksamkeit ohne die dargestellten Nachteile aufweist. Insbesondere ist es daher die Aufgabe wässrige kosmetische Zubereitungen bereit zu stellen, die trotz des Wassergehaltes eine erhöhte Antitranpirant Wirksamkeit durch den Zusatz von aktivierten Aluminiumkomplexsalzen aufweisen.

Gelöst wird das Bündel an Aufgaben durch eine kosmetische Formulierung entsprechend Anspruch 1. Gegenstand der Unteransprüche sind vorteilhafte Ausführungsformen der erfindungsgemäßen Zubereitung. Des weiteren umfasst die Erfindung die Verwendung derartiger Zubereitungen.

Es war überraschend und für den Fachmann nicht vorauszusehen, dass eine kosmetische Formulierung umfassend mindestens eine als Antitranspirant wirksame aktivierte Aluminiumverbindung, mindestens Mandelsäure und Wasser die Bereitstellung einer transparenten und wenig klebrigen kosmetischen Antitranspirantzubereitung ermöglicht.

Durch die Kombination von als Antitranspirant wirksamen aktivierten Aluminiumverbindungen, insbesondere aktiviertes Aluminiumchlorohydrat (AACH) und Mandelsäure, lassen sich wässrige, bevorzugt auch transparente kosmetische Zubereitungen herstellen.

Vorteilhaft weisen diese Zubereitungen keinerlei objektiv als auch subjektiv empfundene Klebrigkeit auf.

Als Antitranspirantwirkstoff lassen sich vorteilhaft aktivierte saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkoniurn-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keiner Weise einschränkend sein:

Aluminium-Salze (der empirischen Summenformel [Al₂(OH)ₘClₙ], wobei m+n=6):
- aktiviertes Aluminiumchlorhydrat [Al₂(OH)₅Cl] x H₂O
   Aktivierte Al-Komplexe: Reach 501 (Reheis), Aloxicoll 51 L
- aktiviertes Aluminiumsesquichlorhydrat [Al₂(OH)_{4.5}Cl_{1.5}] x H₂O
   Aktivierte Al-Komplexe: Reach 301 (Reheis)

Aluminium-Zirkonium-Salze:
- Aluminium/Zirkonium Trichlorhydrex Glycin [Al₄Zr(OH)₁₃Cl₃] x H₂O x Gly
   Standard Al/Zr-Komplexe: Rezal 33GC (Reheis), AZG-7164 (Summit)
- AluminiumlZirkonium Tetrachlorhydrex Glycin [Al₄Zr(OH)₁₂Cl₄] x H₂O x Gly Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
- Aluminium/Zirkonium Pentachlorhydrex Glycin [Al₈Zr(OH)₂₃Cl₅] x H₂O x Gly Standard Al/Zr-Komplexe: Rezal 67 (Reheis), Zirkanal L540, Zirkonal L530 PG (Giulini), Westchlor ZR 80B (Westwood Chemicals)
- Aluminium/Zirkonium Octachlorhydrex Glycin [Al₈Zr(OH)₂₀Cl₈] x H₂O x Gly: Westchlor ZR 82B
- Reach AZP - 908 SUF activated Aluminum Zirkonium Tetrachlorohydrex Gl
- Reach AZZ - 902 SUF activated Aluminum Zirkonium Trichlorohydrex Glyc

Ebenso vorteilhaft können aber auch Glycin-freie Aluminium/Zirkonium-Salze eingesetzt werden.

Die Antitranspirant-Wirkstoffe werden in den erfindungsgemäßen Formulierungen in einer Menge von 1 bis 35 Gew.%, vorzugsweise von 1 bis 20 Gew. %, eingesetzt.

Zusätzlich ist selbstverständlich möglich weitere nicht aktivierte Antitranspirant Wirkstoffe und/oder Deodorantien zu zusetzen.

Die aktivierten Aluminium-Komplexsalze (AACH) zerfallen bekannter weise in Wasser in ihren ursprünglichen Gleichgewichtszustand zurück, so dass in wässrigen Zubereitungen eine erhöhte Wirksamkeit verloren geht.

Ein Einsatz der aktivierten ACH-Typen (AACH) macht bislang daher nur in nichtwäßrigen Systemen einen Sinn, da sonst eine Rückbildung zu der Molekülgrößenverteilung , wie sie in klassischen ACH-Lösungen vorkommt, möglich ist.

Durch den Zusatz an Mandelsäure wird nun überraschenderwiese diese Rückbildung vermieden.

Es wird vermutet, dass eine Komplexbildung, beispielsweise AACH-Mandelsäure, die Ursache für diesen Effekt darstellt.

So könnte die Bildung eines Chelat-Komplex durch Aluminium mit der alpha-Hydroxygruppe und der Säurehydroxygruppe der Mandelsäure unter Freisetzung von Protonen erfolgen. Dieser Komplex ist sehr stabil. Außerdem erklärt die Bindung an diesen beiden Hydroxygruppen, warum bei der Mandelsäure eine erfindungsgemäße Gelierung zu beobachten war.

Zudem können die Phenylreste der Mandelsäure sich über die van-der-Waals-Kräfte aneinander und somit zu einem Gerüst zusammenlagern.

Weiterhin könnten die freigesetzten Protonen den Al-Komplex aufbrechen, wodurch es zur Einlagerung von Wasser in den helixartigen Strukturen des AACH kommen kann.

Entscheidend ist, dass durch die Kombination aus Mandelsäure und aktivierten ACH in wässrigen Medien keinerlei Destruktion der Aktivierung zu beobachten ist.

Die Hydroxyphenylessigsäure oder auch Phenylglykolsäure mit der Formel H₅C₆ -CH(OH) -COOH, C₈H₈O₃ ist bekannt unter dem Namen Mandelsäure. Die Mandelsäure ist gut löslich in Wasser, Alkohol, Ether u. 2-Propanol. Synthetisch erhält man die (±)-Mandelsäure aus Benzaldehyd und Blausäure über das α-Hydroxynitril (Cyanohydrin) und dessen saure Hydrolyse entsprechend Abbildung 1:

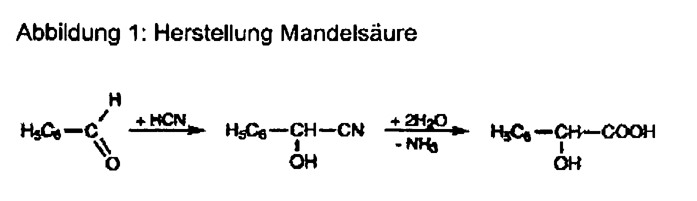

Mittels der Mandelsäure, läßt sich überraschenderweise eine AT-Zubereitung herstellen, die die geforderten Eigenschaften, wie Beibehaltung des aktivierten Zustands, erhöhte Wirksamkeit, Transparenz und geringe Klebrigkeit und darüber hinaus auch die Einstellung einer bestimmten Fließgrenze der Zubereitung ermöglicht. Des weiteren zieht die erfindungsgemäße Formulierung sehr schnell ohne Rückstände zu hinterlassen in die Haut ein.

Die Fließgrenze oder Fließpunkt ist eine Bezeichnung für die kleinste Schubspannung, oberhalb derer ein plastischer Stoff sich rheologisch wie eine Flüssigkeit verhält (DIN 1342-1: 1983-10). Die Bestimmung der Fließgrenze erfolgt durch Aufnahme einer Fließkurve (DIN 53019: 1980-05; DIN 53214: 1982-02). Der erhaltene Wert hängt stark von der Zeitskala (Belastungsrate) ab, die der Messung zugrunde liegt. Dies ist unabhängig davon, ob die Messung mit einem schubspannungs- oder drehzahlgesteuerten Viskosimeter erfolgt. Kurze Zeitskalen (schnelle Belastungen) ergeben in der Regel höhere Werte für die Fließgrenze. Eine zu hohe Fließgrenze kann Ursache von Verlaufstörungen sein. Andererseits lässt sich mit geeignet bemessener Fließgrenze die Neigung der flüssigen Formulierung zum Ablaufen unterdrücken.

Die erfindungsgemäße Zubereitung liegt daher vorteilhaft als Gel- bzw. Hydrogel vor und weist eine Fließgrenze auf, wodurch die Ausbringung und Applikation gegenüber den Zubereitungen aus dem Stand der Technik verbessert ist.

Die erfindungsgemäße Kombination aus AT-Wirkstoff. Mandelsäure und Wasser ermöglicht über einen einzigartigen Verdickungsmechanismus die Herstelltung einer transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist ein angenehmes Hautgefühl aufgrund der fehlenden Klebrigkeit auf.

Mittels der Mandelsäure, und dem AT-Wirkstoff - aktiviertes Aluminium-Salz - läßt sich überraschenderweise ein Hydrogel herstellen, dass die geforderten Eigenschaften, wie Transparenz und geringe Klebrigkeit aufweist. Darüber hinaus zieht die erfindungsgemäße Formulierung sehr schnell ohne Rückstände zu hinterlassen in die Haut ein. Tabelle 1 zeigt den Vergleich verschiedener transparenter Fomullerungen in einem Sensorik-Research-Panel, bestehend aus 8 geschulten Prüfern. Dazu wurden die Proben in definierter Menge auf die Haut aufgetragen und anhand einer Bewertungsskala bewertet (1 = nicht klebrig; 10 = stark klebrig).

**Tabelle 1**

| | **erfindungsgemäßes Beispiel** | **Vergleichsbeispiele** | | |
|---|---|---|---|---|
| | **Transparentes Hydrogel** | **Nano-emulsion** | **Wasser-in-Silikon-Emulsion** | **Wässrig-alkoholische Formulierung** |
| **Einzugsvermögen in Sekunden** | 95 | 179 | 153 | 106 |
| **Klebrigkeit Skala von 1-10** | 3,4 | 5,2 | 6,5 | 5,3 |

Als besonders vorteilhaft hat sich eine Kombination aus Mandelsäure und aktiviertem Aluminium Chlorohydrat gezeigt, wobei das Verhältnis Aluminium Chlorohydrat zu Mandelsäure 15:1 bis 1;1, bevorzugt 12:1 bis 2:1, insbesondere 10:1 bis 2,5:1 ist.

Der Nachweis, dass aktiviertes ACH gegenüber nicht aktiviertem ACH eine bessere Antitranspirant Wirkung aufweist ist bekannt, wie auch das nachstehende Diagramm erläutert.

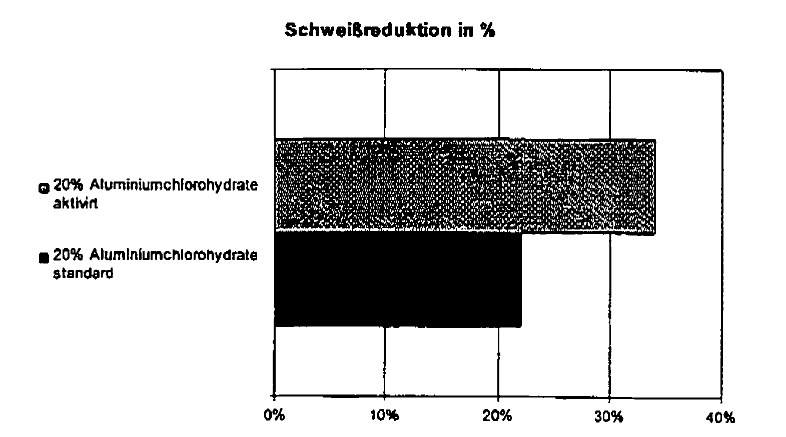

Die bislang bekannten Tests liefen aber nur in wasserfreien Formeln, da bislang davon ausgegangen wurde, dass das AACH in wässrigen Formeln nicht stabil ist.

Somit ist die dargestellte Antitranspirant Wirkung. Schweißreduktion, der erfingdungsgemäß wässrigen Zubereitungen mit denjenigen Zubereitungen vergleichbar, die AACH in nicht wässrigem Milieu enthalten.

Neben den Hydrogelen oder wässrigen Zubereitungen kann es sich bei den erfindungsgemäßen Zubereitungen auch um emulsionsbasierende Zubereitungen handeln.

Vorteilhaft basiert die erfindungsgemäße Zubereitung auf Mikroemulsionen, bevorzugt sind O/W-Mikroemulsionen, insbesondere Mikroemulsionsgelen wie sie in der WO 9815255 und WO 9628132 beansprucht werden, die darin getroffenen Offenbarungen gehören hiermit explizit zum Offenbarungsgehalt der vorliegenden Erfindung.

Die kosmetische Formulierung ist demnach bevorzugt auf Basis von Mikroemulsionsgelen, die a) auf Mikroemulsionen vom Typ Öl-in-Wasser beruhen, welche umfassen
- eine Ölphase, welche im wesentlichen aus schwerflüchtigen Bestandteilen zusammengesetzt ist, und eine Wasserphase
- enthaltend:
- einen oder mehrere polyethoxylierte O/W-Emulgatoren und/oder
- einen oder mehrere polypropoxylierte O/W-Emulgatoren und/oder
- einen oder mehrere polyethoxylierte und polypropoxylierte O/W-Emulgatoren,
- gewünschtenfalls ferner enthaltend einen oder mehrere W/O-Emulgatoren
- einen Emulgatorgehalt kleiner als 20 Gew.-%, bezogen auf das Gesamtgewicht der Emulsion, aufweisend,
- erhältlich auf die Weise, dass ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt,

(b) bei welcher die Tröpfchen der diskontinuierlichen Ölphase durch eine oder mehrere Vernetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

Allerdings problematisch an den in der WO 9815255 und WO 9628132 beschriebenen Mikroemulsionen ist, dass eine definierte Fließgrenze nicht einstellbar war. Diese Aufgabe ist durch die vorliegende Erfindung ebenfalls gelöst worden.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 µm bis ca. 50 µm, Solche "Makroemulsionen" sind, ohne weitere färbende Zusätze, milchig weiß gefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulich weiß gefärbt und undurchsichtig.

Mizellaren und molekularen Lösungen mit Partikeldurchmessern kleiner als ca. 10⁻² µm, ist vorbehalten, klar und transparent zu erscheinen.

Der Tröpfchendurchmesser von transparenten bzw. transluzenten Mikroemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers. Die Viskosität dieser Mikroemulsionen kann mit Hilfe von Assoziatiwerdickern erhöht werden, so dass dann viskose Gele vorliegen.

Die erfindungsgemäße Zubereitung liegt weiterhin vorteilhaft als Gel vor und weist eine Fließgrenze auf, wodurch die Ausbringung und Applikation gegenüber den Zubereitungen aus dem Stand der Technik verbessert ist.

Als Emulgatoren werden neben den aus dem Stand der Technik bekannten insbesondere Fettalkoholethoxylate wie beispielsweise Polyethylenglycol(16)stearylether, Fettsäureethoxylate wie beispielsweise Polyethylenglycol(14)stearat, Polyethylenglycolglycerinfettsäureester wie beispielsweise Polyethylenglycol(15)glyceryllaurat sowie als W/O-Emulgator beispielsweise Glycerylmonostearate verwendet.

Die Ölphase besteht vorzugsweise aus Estern aus gesättigten und ungesättigten, verzweigten und unverzweigten Alkancarbonsäuren oder Alkoholen mit Kettenlängen von 12-25 C-Atomen, wie beispielsweise Octyldodecanol.

Die erfindungsgemäße Kombination aus aktiviertem AT-Wirkstoff, Mandelsäure und Mikroemulsion, bevorzugt die in der WO 9815255 und WO 9628132 offenbarten Mikroemulsionen, ermöglicht über einen einzigartigen Verdickungsmechanismus die Herstelltung einer transparenten kosmetischen Zubereitung. Der Anwender hat somit erstmalig eine wasserklare und dennoch überaus wirksame Zubereitung zur Hand. Die erfindungsgemäße Zubereitung ist in Gelform bequem zu applizieren und weist ein angenehmes Hautgefühl aufgrund der fehlenden Klebrigkeit auf.

Durch die Kombination von aktivierten Antitranspirantwirkstoffen und Mandelsäure in O/W-Mikromemulsionen lassen sich transparente kosmetische Formulierungen herstellen, die verminderte bzw. keinerlei objektiv als auch subjektiv empfundene Klebrigkeit aufweisen und vor allem Zubereitungen, die keinerlei Einbussen des Aktivitätsverlustes aufzeigen.

| | **Mikroemulsion mit Mandelsäure** | **Mikroemulsion mit Assozlativverdicker** |
|---|---|---|
| **Einzugsvermögen in Sekunden** | 123 | 149 |
| **Klebrigkeit Skala von 1-10** | 4,1 | 5,5 |

Vorteilhaft können erfindungsgemäßen Zubereitungen Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile. Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin). 3,4,4'-Trichlorcarbanilid, quaternäre Ammoniumverbindungen, Nelkenöl, Minzöl, Thymianöl, Triethylcitrat, Farnesol (3.7,11-Trimethyl-2,6,10-dodecatriën-1-ol) sowie die in den DE 37 40 186, DE 39 38 140. DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Menge der Desodorantien (eine oder mehrere Verbindungen) in den Zubereitungen beträgt vorzugsweise 0,01 bis 10 Gew,%, bevorzugt 0,05 bis 5 Gew.% bezogen auf das Gesamtgewicht der Zubereitung.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter. Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Die Herstellung der erfindungsgemäß transparenten gelförmigen Zubereitung erfolgt vorteilhaft durch Lösen der Mandelsäure in Wasser. Parallel wird der AT-Wirkstoffe, insbesondere das aktivierte Aluminiumchlorohydrate, in Wasser gelöst. Anschließend werden beide Phasen vereint und 1 h gerührt.

Zur Applikation der Zubereitung lassen sich herkömmliche Packmittel für Deodorantien und/oder Antitransipirantien verwenden, z. B. Stiftdispenser, Geldispenser, Tuben und Roller.

Die erfindungsgemäße Formulierung umfasst Mandelsäure bevorzugt in einer Menge von 0,1 bis 10 Gew.%, vorzugsweise von 0,1 bis 8 Gew.% bezogen auf die Gesamtmasse der Formulierung.

Die Formulierung weist bevorzugt eine definierte Fließgrenze von 40 bis 120 Pa (mittels Schubspannungszeitrampe (40 Pa/min; 25°C)) auf.

Die erfindungsgemäße kosmetischen Formulierung ist zur Auftragung auf die menschliche Haut geeignet.

Die erfindungsgemäße kosmetischen Formulierung ist als Antitranspirant zu verwenden, insbesondere zur Herstellung eines transparenten Antitranspirant-Hydrogels.

Die Kombination aus Mandelsäure und antitranspirant wirksamer aktivierter Aluminiumverbindung ist zur Herstellung einer wässrigen Antitranspirantzubereitung geeignet.

Die folgenden Angaben sind in Gewichtsprozent bezogen auf die Gesamtmasse der Zubereitung.

| Beispiele | **1** | **2** | **3** |
|---|---|---|---|
| aktiviertes Aluminum Chlorhydrat | 5 | 10 | 10 |
| Mandelsäure | 1,4 | 1,8 | 2 |
| Natriumcitrat | - | - | 1 |
| Wasser | 93,6 | 88,2 | 87 |
| Summe | 100 | 100 | 100 |

| Beispiele | **4** | **5** | **6** |
|---|---|---|---|
| Glyceryl Isostearate | 2,6 | 2,5 | 2,5 |
| Isoceteth-20 | 5 | 5 | 5 |
| PEG-150 Distearat | 1 | 1,5 | 0,7 |
| Dicaprylyl Ether | 5 | 5 | 5 |
| Mandelic Acid | 1,5 | 1,5 | 2 |
| aktiviertes Aluminum Chlorohydrate | 10 | 10 | 10 |
| Perfuem | 1 | 1 | 1 |
| Butylene Glycol | 3 | - | 3 |
| Methylparaben | 0,2 | 0,2 | - |
| Wasser | 70,7 | 73,3 | 70,8 |
| Summe | 100 | 100 | 100 |

## Patentansprüche

1. Kosmetische Formulierung umfassend mindestens eine antitranspirant wirksame aktivierte Aluminiumverbindung, mindestens eine α-Hydroxycarbonsäure und Wasser, **dadurch gekennzeichnet, dass** als Hydroxycarbonsäure Mandelsäure gewählt wird.

2. Formulierung nach Anspruch 1, **dadurch gekennzeichnet, dass** der Antitranspirant-Wirkstoff aus der Gruppe der aktivierten Aluminium-Salze, bevorzugt aktiviertes Aluminium-Chlorohydrat, gewählt wird.

3. Formulierung nach einem der voranstehenden Ansprüche, **dadurch gekennzeichnet, dass** das Verhältnis aktiviertes Aluminium chlorohydrat zu Mandelsäure im Bereich 15:1 bis 1:1, bevorzugt 12:1 bis 2:1, insbesondere 10 : 1 bis 2:1, gewählt wird.

4. Formulierung nach einem der voranstehenden Ansprüche, **dadurch gekennzeichnet, dass** der Antitranspirant-Wirkstoff in einer Menge von 1 bis 35 Gew.%, bevorzugt von 1 bis 20 Gew.% bezogen auf die Gesamtmasse der Formulierung, eingesetzt wird.

5. Formulierung nach einem der vorherigen Ansprüche, **dadurch gekennzeichnet, dass** es eine O/W-Mikroemulsion ist.

6. Formulierung nach Anspruch 5, **dadurch gekennzeichnet, dass** es ein Mikroemulsionsgel ist.

7. Kosmetische Formulierung nach Anspruch 6 auf Basis von Mikroemulsionsgelen,
a) beruhend auf Mikroemulsionen vom Typ Öl-in-Wasser, welche umfassen
- eine Ölphase, welche im wesentlichen aus schwerflüchtigen Bestandteilen zusammengesetzt ist, und eine Wasserphase
- enthaltend:
- einen oder mehrere polyethoxylierte O/W-Emulgatoren und/oder
- einen oder mehrere polypropoxylierte O/W-Emulgatoren und/oder
- einen oder mehrere polyethoxylierte und polypropoxylierte O/W-Emulgatoren,
- gegebenenfalls ferner enthaltend einen oder mehrere W/O-Emulgatoren
- einen Emulgatorgehalt kleiner als 20 Gew.-%, bezogen auf das Gesamtgewicht der Emulsion, aufweisend,
- erhältlich auf die Weise, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

8. Formulierung nach einem der voranstehenden Ansprüche, **dadurch gekennzeichnet, dass** die Formulierung eine definierte Fließgrenze aufweist.

## Claims

1. Cosmetic formulation comprising at least one antiperspirant activated aluminium compound, at least one α-hydroxycarboxylic acid and water, **characterized in that** the hydroxycarboxylic acid chosen is mandelic acid.

2. Formulation according to Claim 1, **characterized in that** the antiperspirant active ingredient is chosen from the group of activated aluminium salts, preferably activated aluminium chlorohydrate.

3. Formulation according to one of the preceding claims, **characterized in that** the ratio of activated aluminium chlorohydrate to mandelic acid is chosen in the range 15:1 to 1:1, preferably 12:1 to 2:1, in particular 10:1 to 2:1.

4. Formulation according to one of the preceding claims, **characterized in that** the antiperspirant active ingredient is used in an amount of from 1 to 35% by weight, preferably from 1 to 20% by weight, based on the total mass of the formulation.

5. Formulation according to one of the preceding claims, **characterized in that** it is an O/W microemulsion.

6. Formulation according to Claim 5, **characterized in that** it is a microemulsion gel.

7. Cosmetic formulation according to Claim 6 based on microemulsion gels,
a) based on microemulsions of the oil-in-water type which comprise
- an oil phase which is essentially composed of difficultly volatile constituents, and a water phase
- comprising:
- one or more polyethoxylated O/W emulsifiers and/or
- ane or more polypropoxylated O/W emulsifiers and/or
- one or more polyethoxylated and polypropoxylated O/W emulsifiers,
- optionally also comprising one or more W/O emulsifiers
- having an emulsifier content of less than 20% by weight, based on the total weight of the emulsion,
- obtainable by bringing a mixture of the base components, comprising water phase, oil phase, one or more of the O/W emulsifiers according to the invention, if desired one or more W/O emulsifiers, and if desired further auxiliaries, additives and/or active ingredients, to a temperature within or above the phase inversion temperature range, and subsequently cooling to room temperature
(b) in which the droplets of the discontinuous oil phase are joined together by one or more crosslinker substances whose molecules are **characterized by** at least one hydrophilic region which has a breadth suitable for bridging the distance between the microemulsion droplets, and by at least one hydrophobic region which is able to enter into hydrophobic interaction with the microemulsion droplets.

8. Formulation according to one of the preceding claims, **characterized in that** the formulation has a defined yield point.

## Revendications

1. Composition cosmétique comprenant au moins un composé d'aluminium activé, à effet antisudoral, au moins un acide α-hydroxycarboxylique et de l'eau, **caractérisée en ce qu'**on choisit comme acide hydroxycarboxylique l'acide mandélique.

2. composition selon la revendication 1, **caractérisée en ce qu'**on choisit la substance active antisudorale dans le groupe des sels d'aluminium activés, de préférence le chlorhydrate d'aluminium activé.

3. Composition selon l'une quelconque des revendications précédentes, **caractérisée en ce que** le rapport du chlorhydrate d'aluminium activé à l'acide mandélique est choisi dans la plage de 15:1 à 1:1, de préférence de 12:1 à 2:1, en particulier de 10:1 à 2:1.

4. Composition selon l'une quelconque des revendications précédentes, **caractérisée en ce que** la substance active antisudorale est utilisée en une quantité de 1 à 35 % en poids, de préférence de 1 à 20 % en poids, par rapport à la masse totale de la composition.

5. Composition selon l'une quelconque des revendications précédentes, **caractérisée en ce qu'**elle est une microémulsion H/E.

6. Composition selon la revendication 5, **caractérisée en ce qu'**elle est un gel de microémulsion.

7. Composition cosmétique selon la revendication 6 à base de gels de microémulsions,
a) à base de microémulsions du type huile-dans-eau,
- comprenant une phase huileuse, qui est essentiellement composée de composants peu volatils, et une phase aqueuse
- contenant :
- un ou plusieurs émulsifiants H/E polyéthoxylés et/ou
- un ou plusieurs émulsifiants H/E polypropoxylés et/ou
- un ou plusieurs émulsifiants H/E polyéthoxylés et polypropoxylés,
- éventuellement contenant en outre un ou plusieurs émulsifiants E/H,
- ayant une teneur en émulsifiants inférieure à 20 % en poids, par rapport au poids total de l'émulsion,
- pouvant être obtenues en portant à une température dans ou au-dessus de la plage de température d'inversion de phase un mélange des composants de base, comprenant phase aqueuse, phase huileuse, un ou plusieurs des émulsifiants H/E selon l'invention, éventuellement un ou plusieurs émulsifiants E/H, ainsi que, si on le désire, d'autres adjuvants, additifs et/ou substances actives, et ensuite en le refroidissant jusqu'à la température ambiante,
b) dans laquelle les gouttelettes de la phase huileuse discontinue sont liées les unes aux autres par une ou plusieurs substances de réticulation, dont les molécules se distinguent par au moins une région hydrophile qui présente une extension qui est appropriée à ponter la distance des gouttelettes de microémulsion entre elles, et par au moins une région hydrophobe qui est capable d'entrer en interaction hydrophobe avec les gouttelettes de microémulsion.

8. Composition selon l'une quelconque des revendications précédentes, **caractérisée en ce que** la composition présente une limite d'écoulement définie.
